# EUROPEAN PATENT SPECIFICATION

(11) **EP 4 164 741 B1**
(45) Date of publication and mention of the grant of the patent: **25.11.2026**
(21) Application number: 21733404.4
(22) Date of filing: 10.06.2021
(51) Int. Cl.: A61N 5/10

(54) **METHOD AND APPARATUS TO FACILITATE ADMINISTERING THERAPEUTIC RADIATION TO A PATIENT**
VERFAHREN UND VORRICHTUNG ZUR ERLEICHTERUNG DER VERABREICHUNG VON THERAPEUTISCHER STRAHLUNG AN EINEN PATIENTEN
PROCÉDÉ ET APPAREIL POUR FACILITER L'ADMINISTRATION D'UN RAYONNEMENT THÉRAPEUTIQUE À UN PATIENT

(30) Priority: 11.06.2020 US 202016898608
(43) Date of publication of application: 19.04.2023
(73) Proprietor: Siemens Healthineers International AG, 6312 Steinhausen (CH)
(72) Inventor: BASIRI, Shahab, 02570 Siuntio (FI); KUUSELA, Esa, 02320 Espoo (FI); CZEIZLER, Elena, 00390 Helsinki (FI); HAKALA, Mikko Oskari, 05200 Rajamaki (FI)
(74) Representative: Foster, Mark Charles
(86) International application number: PCT/EP2021/065672
(87) International publication number: WO 2021/250188

(56) References cited:
- US-A1- 2019 333 623

## Description

### Technical Field

These teachings relate generally to treating a patient's planning target volume with radiation pursuant to a radiation treatment plan and more particularly to developing therapeutic radiation delivery field geometry information for a particular patient.

### Background

The use of radiation to treat medical conditions comprises a known area of prior art endeavor. For example, radiation therapy comprises an important component of many treatment plans for reducing or eliminating unwanted tumors. Unfortunately, applied radiation does not inherently discriminate between unwanted materials and adjacent tissues, organs, or the like that are desired or even critical to continued survival of the patient. As a result, radiation is ordinarily applied in a carefully administered manner to at least attempt to restrict the radiation to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often generated through a so-called optimization process. As used herein, "optimization" will be understood to refer to improving a candidate treatment plan without necessarily ensuring that the optimized result is, in fact, the singular best solution. Such optimization often includes automatically adjusting one or more treatment parameters (often while observing one or more corresponding limits in these regards) and mathematically calculating a likely corresponding treatment result to identify a given set of treatment parameters that represent a good compromise between the desired therapeutic result and avoidance of undesired collateral effects. Further, radiation treatment plans can be predicted using neural networks. For example, US 2019/333623 A1 discloses a method wherein a generative adversarial network, GAN, using patient related information and radiotherapy related information as input, generates a treatment plan.

Determining optimal beam delivery geometry (containing, for example, gantry angles, potential couch positions, and collimator positions) is an important but non-trivial step in radiation therapy treatment planning. Such planning typically relies on guidelines, templates, and the expertise of the planner. Unfortunately, defining field geometry using such tools as static templates fails to take into account specific patient geometry and hence can yield unsatisfactory results.

In some cases, at least some aspects of the field geometry selection (such as field delivery directions (i.e., the distribution of gantry angles in coplanar treatments)) can be accomplished using coarse optimization algorithms (such as a beam angle optimizer or trajectory optimizer), but even this approach is typically based on hand-crafted restrictions. In addition, some prior art approaches can reach solutions that are inappropriately distant from commonly used field geometries. Further complicating matters is the common practice of classifying cases as belonging to a particular class, such as left- or right-hand-sided or full-arc treatment.

### Summary

According to one aspect of the invention, there is provided an apparatus to facilitate generating therapeutic radiation delivery field geometry information for a particular patient, as defined in claim 1. Optional features are specified in the claims dependent on claim 1.

According to another aspect of the invention, there is provided a method to facilitate generating therapeutic radiation delivery field geometry information for a particular patient, as defined in claim 9.

Optional features are specified in the claims dependent on claim 9. The claimed method does not include applying the generated therapeutic radiation delivery field geometry to the particular patient.

### Brief Description of the Drawings

The above needs are at least partially met through provision of the method and apparatus to develop therapeutic radiation delivery field geometry information for a particular patient described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a block diagram as configured in accordance with various embodiments of these teachings; and
FIG. 4 comprises a graph.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### Detailed Description

Generally speaking, these various embodiments serve to facilitate providing a radiation treatment plan to administer therapeutic radiation to a patient via a particular radiation treatment platform by automatically generating therapeutic radiation delivery field geometry as a function, at least in part, of that patient.

By one approach, these teachings provide for having a control circuit access information corresponding to patient geometry information for a particular patient. The control circuit then provides that information, along with at least one variable that is unrelated to that particular patient, as input to a field geometry generator. By one approach the field geometry generator comprises a neural network trained in a conditional generative adversarial networks (GAN) framework as a function of previously-developed field geometry solutions for a plurality of different patients. In such a case the information corresponding to the patient geometry information for the particular patient can serve as conditional input to the neural network. So configured, the control circuit can then process the foregoing input using the field geometry generator to thereby generate the therapeutic radiation delivery field geometry for the particular patient.

By one approach the aforementioned patient geometry information for the particular patient comprises images. If desired, the patient geometry information for the particular patient can comprise only images. Examples in these regards include, but are not limited to, images that depict at least one segmented and contoured organ-at-risk and at least one segmented and contoured planning target volume.

By one approach, the aforementioned variable that is unrelated to the particular patient comprises a vector of random numerical input.

The generated therapeutic radiation delivery field geometry may comprise, for example, field delivery directions. The control circuit may utilize these field delivery directions when optimizing a radiation treatment plan that can be used to administer therapeutic radiation to this particular patient.

By one approach these teachings will accommodate preprocessing the aforementioned patient geometry information for the particular patient to yield the information that is provided as input to the field geometry generator. This preprocessing may comprise, for example, reducing the dimensionality of the patient geometry information. Such an approach can be particularly helpful when the patient geometry information comprises, at least in part, a multidimensional numerical representation corresponding to an aggregation of different modalities of informational content such as, but not limited to, both imagery and non-imagery content.

So configured, these teachings facilitate using and leveraging local data that is already available at a particular site (such as a treatment clinic). In particular, local data regarding patient geometries for a variety of patients and the corresponding approved field geometries can be leveraged to learn the distribution of the previously used beam delivery directions, especially the utilized gantry angles, conditioned on the patient geometry. These teachings accordingly support using existing data when selecting a suitable field geometry for a new patient.

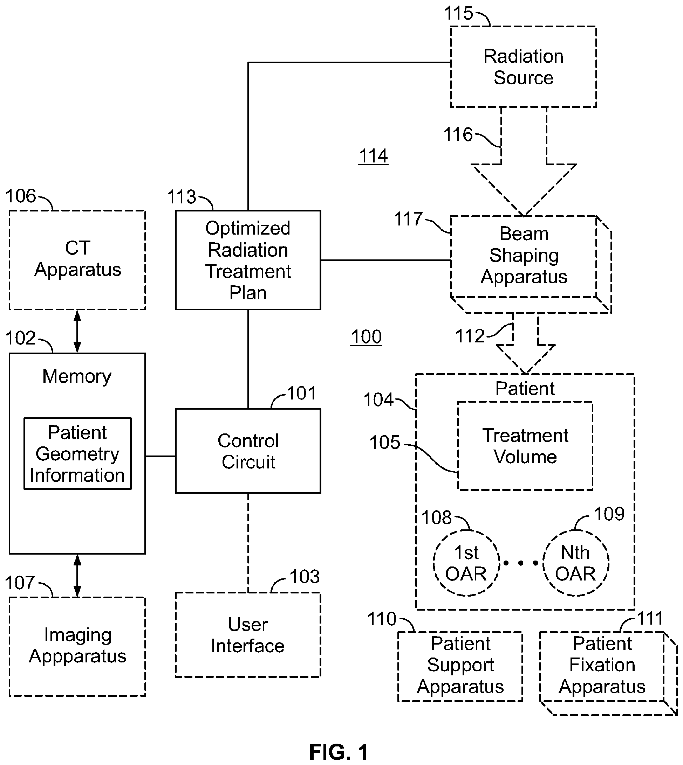

These and other benefits may become clearer upon making a thorough review and study of the following detailed description. Referring now to the drawings, and in particular to FIG. 1, an illustrative apparatus 100 that is compatible with many of these teachings will now be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101).

In addition to the aforementioned patient geometry information for a particular patient, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).) For example, the computer instructions can serve to configure the control circuit 101 to act as a field geometry generator that comprises a neural network trained in a conditional generative adversarial network framework as described herein.

In this example the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of the patient geometry information described herein.

In this illustrative example the control circuit 101 may be configured to ultimately output an optimized radiation treatment plan 113. This radiation treatment plan 113 typically comprises specified values for each of a variety of treatment-platform parameters during each of a plurality of sequential fields. In this case the radiation treatment plan 113 is generated through an optimization process. Various automated optimization processes specifically configured to generate such a radiation treatment plan are known in the art. As the present teachings are not overly sensitive to any particular selections in these regards, further elaboration in these regards is not provided here except where particularly relevant to the details of this description.

By one approach the control circuit 101 can operably couple to a radiation treatment platform 114 that is configured to deliver therapeutic radiation 112 to a treatment volume 105 of a corresponding patient 104 in accordance with the optimized radiation treatment plan 113 that also seeks to minimize such exposure to one or more of the patient's organs-at-risk 108, 109. These teachings are generally applicable for use with any of a wide variety of radiation treatment platforms. In a typical application setting the radiation treatment platform 114 will include radiation source 115. The radiation source 115 can comprise, for example, a radio-frequency (RF) linear particle accelerator-based (linac-based) x-ray source, such as the Varian Linatron M9. The linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons. A typical radiation treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more beam-shaping apparatuses 117 (such as jaws, multi-leaf collimators, and so forth) to provide selective beam shaping and/or beam modulation as desired. As the foregoing elements and systems are well understood in the art, further elaboration in these regards is not provided here except where otherwise relevant to the description.

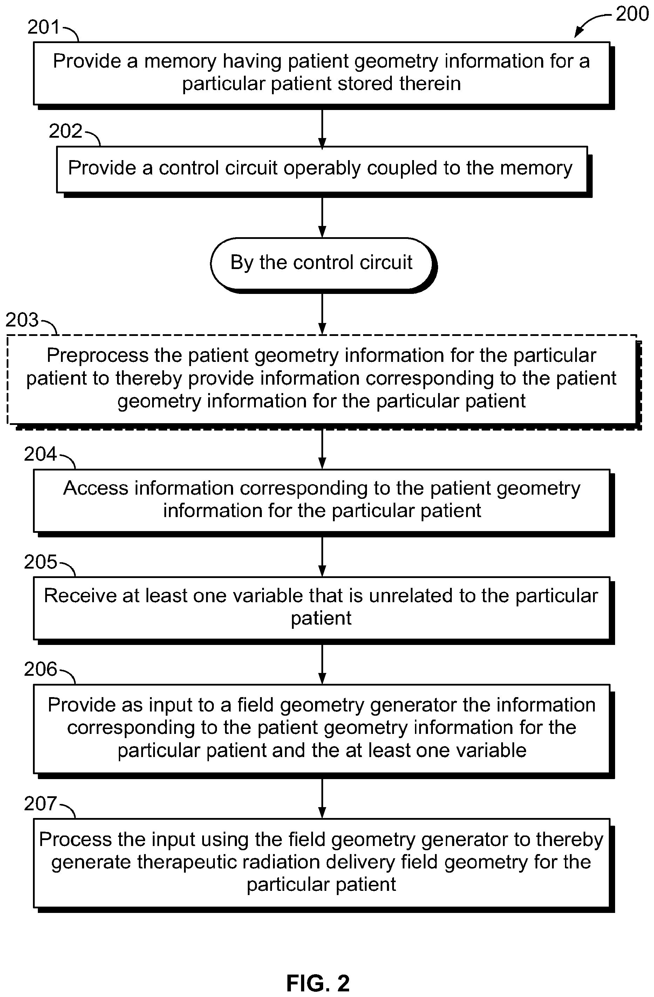

Referring now to FIG. 2, a process 200 that can be carried out, for example, by the above-described control circuit 101 will now be presented.

At block 201, this process 200 provides a memory (such as the above-described memory 102) that has patient geometry information for a particular patient stored therein. Patient geometry information can comprise information regarding sizes, shapes, dimensionality, and the relative distances between and amongst one or more planning target volumes (such as tumors) and/or organs-at-risk for a particular patient. Such information can be provided for any of a plurality of different fields of view for such subjects.

By one approach the patient geometry information comprises images. Examples in these regards include, but are not limited to, images that depict at least one segmented and contoured planning target volume and/or organ-at-risk for the patient. If desired, the patient geometry information for the particular patient comprises only images. (Contouring refers to specifying the outline of individual organs, tissues, or other anatomical structures and artifacts of the patient such as, but not limited to, target treatment volumes and organs-at-risk, while segmentation refers to identifying discrete patient structures including, but not limited to, target treatment volumes and particular organs-at-risk.)

Block 202 of this process 200 comprises providing a control circuit that operably couples to the aforementioned memory, such as the above-described control circuit 101. For the sake of clarity and a simple illustrative example, the remainder of this description presumes that the remaining steps of this process 200 are carried out by the provided control circuit.

Before using the above-described patient geometry information to generate therapeutic radiation delivery field geometry for the particular patient, this process will optionally accommodate, as illustrated at optional block 203, preprocessing the patient geometry information for the particular patient to thereby provide information corresponding to the patient geometry information for the particular patient that can serve as input information as described below. Preprocessing the patient geometry information can comprise, at least in part, reducing the dimensionality of the patient geometry information. This reduction in dimensionality can be particularly beneficial when the patient geometry information comprises, at least in part, a multidimensional numerical representation corresponding to an aggregation of different modalities of informational content. The presence of different modalities of informational content can occur, for example, when the informational content includes both imagery and non-imagery content. (Further description is provided below regarding such preprocessing.)

At block 204, the control circuit 101 accesses information corresponding to the patient geometry information for the particular patient. By one approach this can comprise directly accessing and utilizing the patient geometry information that is stored in the aforementioned memory 102. By another approach, this can comprise, at least in part, accessing patient geometry information that has been preprocessed as described above.

At block 205, the control circuit 101 also receives at least one variable that is unrelated to the particular patient. By one approach this at least one variable can comprise a vector of random (or pseudorandom) numerical input.

At block 206, the control circuit 101 then provides as input to a field geometry generator the aforementioned information corresponding to the patient geometry information for the particular patient as well as the aforementioned at least one variable. In this example the control circuit 101 serves as this field geometry generator by being configured as a neural network trained in a conditional generative adversarial networks (GAN) framework as a function of previously-developed field geometry solutions for a plurality of different patients.

Those skilled in the art will understand that a GAN is a class of machine learning frameworks that place two neural networks in a contested setting with one another. Given a particular training set, this methodology learns to generate new data with the same statistics as the training set. A GAN typically comprises a generative network that generates candidates and a discriminative network that evaluates the candidates generated by the generative network. The generative network's primary training objective is to increase the error rate of the discriminative network by providing the discriminative network with newly generated candidates that the discriminative network identifies as being part of the true data distribution.

By one approach these teachings will accommodate configuring the control circuit 101 as a conditional GAN. In such a case the information corresponding to the patient geometry information for the particular patient serves as a conditional input to the neural network.

At block 207, and acting as a field geometry generator, the control circuit 101 processes the foregoing input to thereby automatically generate a therapeutic radiation delivery field geometry for the particular patient. By one approach the generated therapeutic radiation delivery field geometry can comprise such parameters as particular field delivery directions (such as specific gantry angles from which radiation is momentarily administered to the patient). These teachings are flexible in practice, however, and will accommodate other approaches if desired. For example, by one approach the field delivery directions may already be fixed and the field geometry generator instead generates other field geometry attributes such as collimator settings.

Accordingly, these teachings will accommodate configuring the control circuit 101 as a generator neural network that is trained in a conditional GAN framework, where the patient geometry is the specific conditional input. By one approach, these teachings can present a data-driven approach where the field geometry generator is trained to produce candidate field geometries based solely on patient geometry (aside from the use of random variables).

This therapeutic radiation delivery field geometry can then be utilized when optimizing a radiation treatment plan. These teachings will also then accommodate using the resultant optimized radiation treatment plan that is based upon the automatically generated therapeutic radiation delivery field geometry in conjunction with a particular radiation treatment platform to administer therapeutic radiation to the particular patient.

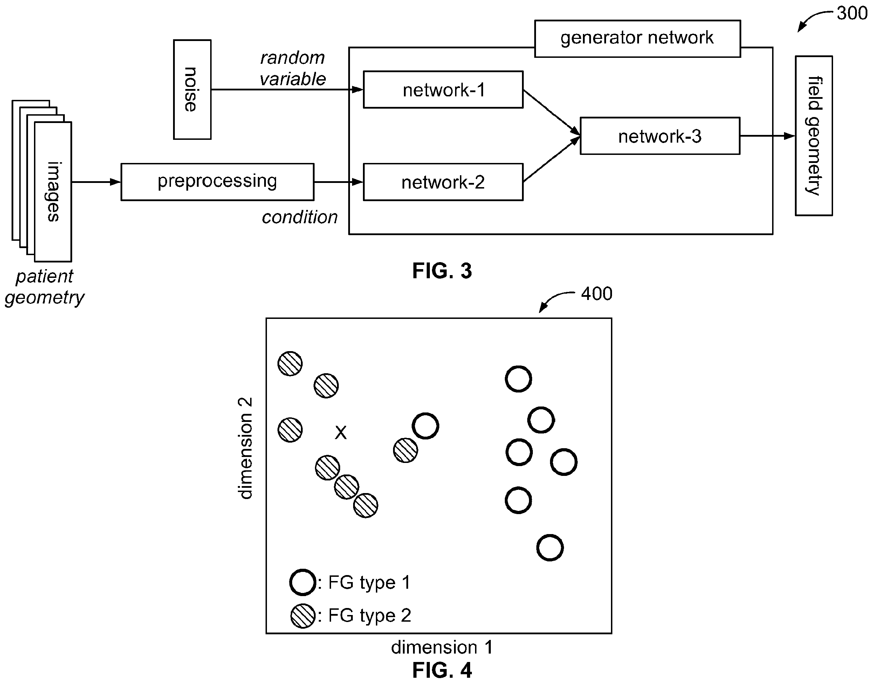

Referring now to FIG. 3, a more specific example in the foregoing regards will be provided. It will be understood that the details of this example are offered for the sake of illustration and are not intended to suggest any particular limitations with respect to these teachings.

In this example, a generative model (generator) 300 is trained in the conditional GAN framework, in which a generator and a discriminator network play a two-player game, where both have their own respective loss functions to be minimized. The inputs in GAN training are patient geometries as conditional labels (denoted x), field geometries (denoted y), and a random vector (denoted z). The solution of the training corresponds to a saddle point in terms of both networks' losses. As the result of training, the generator learns a mapping G: (x, z) --> y. In other words, in this unsupervised machine learning context, the generator learns implicitly an approximation to the density distribution of the underlying field geometries conditioned on the patient geometry, pdata(y|x). The architectures of the generator and discriminator include convolutional layers in this example. When the generator is used for inference with unseen patient geometries, the generator can output samples from the learned distribution of field geometries.

By one approach, a preprocessing step is performed on the patient geometry (comprising, for example, planning computed tomography images with segmented organs and planning target volumes). This preprocessing can comprise projecting the patient geometry imagery to two-dimensional images corresponding to different beam's eye view angles and then downsampling the two-dimensional images to lower resolution.

So configured, these teachings offer a solution that is fully data-driven. In previous solutions, existing clinical knowledge and data have not been much leveraged to create field delivery directions based on patient geometry in a systematic, automated fashion. Use of the trained generator is fast (field geometry candidates can be generated in only a few seconds for preprocessed patient geometry). If the dataset that has been used to train the generator has distinctly different classes (such as left- or right-handed side treatments), the output of the generator is similarly expected to fall into one of these classes, and in this way the generator performs implicitly the selection of the treatment class for a new patient. Those skilled in the art will further appreciate that the workings of this approach can be easily updated (that is, retrained if and as more clinical data becomes available) and deployed as a standard neural network machine learning model.

It will be further appreciated that the field geometry generator described herein readily supports both IMRT and VMAT planning techniques.

Additional details will now be provided as regards the above-referenced preprocessing activity of block 203 of the above-described process 200. In fact, such preprocessing can be useful as part of other related processes if desired.

The patient data that a given clinic or other treatment facility possesses (such as planning CT images with segmented organs and planning treatment volumes, approved radiation treatment plans, patient history data, outcome data, and so forth) can be aggregated in many ways. Such aggregated data forms a unique multidimensional numerical representation of each individual patient (denoted herein generically as patient data Pi for patient i). This representation belongs to the set P of representations of all the patients for which data exist at the same facility. Note that the corresponding dimensionality of such information can be relatively large. For example, in an application setting that includes segmented CT images or some transformation thereof in the numerical representation, the dimension of Pi becomes easily tens of thousands or more (given that a single CT image can be of resolution 256 x 256).

With the foregoing in mind, appropriate preprocessing allows one to work in a low-dimensional space while still retaining a unique representation of the patient.

The applicant has determined that preprocessing that provides dimensionality reduction can comprise a key component in tasks that relate to field geometry selection in radiation therapy treatment planning. By one approach such preprocessing allows the field geometry selection to be done efficiently in a lower-dimensional space of the patient representation. The latter, in turn, allows a faster comparison of a new patient's case to the reference set (which represents previous patients of the treatment facility) for subsequent case analysis and field geometry selection.

By one approach, such preprocessing can begin with accessing heterogeneous raw clinical data for at least a plurality (or even all) of the patients for a given treatment facility such as a particular clinic. The preprocessing can then provide for task-specific aggregation of data and forming a multidimensional numerical representation (thereby forming the aforementioned representations Pi) followed by corresponding dimensionality reduction to thereby form reduced-dimensionality representations denoted here as pi.

A field geometry selection task can then be performed using representations pi to thereby leverage the reduced-dimensionality representations yielded by the preprocessing activity. The selected field geometry can then be utilized as desired. This can comprise, for example, providing a visual representation of that content to a user via the aforementioned user interface 103 and/or by automatically utilizing such information when optimizing a radiation treatment plan.

Dimensionality reduction of the patient data can be performed by any chosen method. By one approach, and depending on the heterogeneity of the starting clinical data, hierarchical categories may be constructed prior to the dimensionality reduction. By one approach dimensionality reduction can be accomplished via any of principal component analysis (PCA), kernel PCA, non-negative matrix factorization, t-distributed stochastic neighbor embedding, or autoencoders, to note but a few examples.

Various tasks related to the field geometry selection can be solved when leveraging these teachings. For a new patient, the tasks may include, but are not limited to, (i) finding field geometry class solutions (as illustrated below with reference to FIG. 4), (ii) proposing one or more field geometries, and (iii) finding patient cases that likely require extra care in treatment planning (for example, borderline cases or outliers with respect to previously treated patients). For existing reference patient data, the tasks may include, but are not limited to, (i) clarifying the level of heterogeneity and variability in the treatment planning procedures and (ii) developing evidence that there may be hidden variables/influences that are affecting the field geometry selection.

By one approach, nearest-neighbor solutions can be sought, and other data analyses performed by any of a variety of different methods (including, for example, the simplest k-nearest neighbor solutions and clustering analyses).

These teachings are highly flexible in practice. By one approach, for example, the input may be segmented patient geometry and the task is to search for the most typical coplanar IMRT field geometries based on previously used field geometries. In this realization, the patient geometries may first be transformed by projecting a stack of segmented organs and planning treatment volumes to the isocenter plane, as seen from different gantry angles, to form the multidimensional representation. Subsequently, dimensionality reduction is performed for examples by principal component analysis, and one or more nearest-neighbor instances are searched from the reference patients. These teachings return as a solution the field geometries that were used for the nearest-neighbor patients.

If desired, outlier detection can be implemented by measuring the distance of any new patient from the previous patients in the dimensionally reduced space.

It will also be appreciated that, as exemplified above, these teachings are not limited only to field geometry selection but are a step that can be used together with other data-driven approaches in many tasks of the radiation therapy treatment planning workflow.

Such approaches are inherently data-driven (in particular, knowledge based), automatic, and provides numerical support for field geometry selection (for example, via distance metrics in the reduced space). Various algorithms utilized in the foregoing approach can be tested and tuned according to the specific task and the needs of the application setting. Visualization of the individual new patient data with respect to any reference data can also be easily accomplished in two dimensions or three dimensions using such dimensionality reduction.

FIG. 4 presents a graph 400 that illustrates an example of using dimensionality reduction to visualize a patient dataset and finding the probable field geometry class solution. The positions of the spheres in this graph 400 correspond to the 2-dimensional representation of the patient geometrical data. The solid circles and open circles correspond to different field geometry selections in the dataset. The unseen patient geometry data (denoted by the letter X) in this illustrative example falls close to field geometry (FG) type 2.

The invention is defined in the claims. Other embodiments, examples, items etc. and in particular method for delivery radiotherapy to a patient are not a part of the invention.

## Claims

1. An apparatus to facilitate generating therapeutic radiation delivery field geometry information for a particular patient, the apparatus comprising:
a memory having patient geometry information for the particular patient stored therein;
a control circuit operably coupled to the memory and configured to:
- access information corresponding to the patient geometry information for the particular patient;
- receive at least one variable that is unrelated to the particular patient;
- provide as input to a field geometry generator the information corresponding to the patient geometry information for the particular patient and the at least one variable, wherein the field geometry generator comprises a neural network trained in a conditional generative adversarial networks, GAN, framework as a function of previously-developed field geometry solutions for a plurality of different patients and wherein the information corresponding to the patient geometry information for the particular patient serves as conditional input to the neural network;
- process the input using the field geometry generator to thereby generate the therapeutic radiation delivery field geometry for the particular patient.

2. The apparatus of claim 1 wherein the patient geometry information for the particular patient comprises images;
and optionally:
wherein the patient geometry information for the particular patient comprises only images.

3. The apparatus of claim 1 or 2 wherein the patient geometry information for the particular patient comprises images depicting at least one segmented and contoured organ-at-risk and at least one segmented and contoured planning target volume.

4. The apparatus of any one of claims 1 to 3 wherein the at least one variable that is unrelated to the particular patient comprises a vector of random numerical input.

5. The apparatus of any one of claims 1 to 4 wherein the therapeutic radiation delivery field geometry that is generated for the particular patient comprises, at least in part, field delivery directions.

6. The apparatus of any one of claims 1 to 5 wherein the control circuit is further configured to:
preprocess the patient geometry information for the particular patient to thereby provide the information corresponding to the patient geometry information for the particular patient.

7. The apparatus of claim 6 wherein the control circuit is configured to preprocess the patient geometry information, at least in part, by reducing dimensionality of the patient geometry information.

8. The apparatus of claim 7 wherein the patient geometry information comprises, at least in part, a multidimensional numerical representation corresponding to an aggregation of different modalities of informational content;
and optionally:
wherein the aggregation of different modalities of informational content include, but are not limited to, imagery and non-imagery content.

9. A method to facilitate generating therapeutic radiation delivery field geometry information for a particular patient, the method comprising:
providing a memory having patient geometry information for the particular patient stored therein;
providing a control circuit operably coupled to the memory; by the control circuit:
accessing information corresponding to the patient geometry information for the particular patient;
receiving at least one variable that is unrelated to the particular patient; providing as input to a field geometry generator the information corresponding to the patient geometry information for the particular patient and the at least one variable, wherein the field geometry generator comprises a neural network trained in a conditional generative adversarial networks, GAN, framework as a function of previously-developed field geometry solutions for a plurality of different patients and wherein the information corresponding to the patient geometry information for the particular patient serves as conditional input to the neural network;
processing the input using the field geometry generator to thereby generate the therapeutic radiation delivery field geometry for the particular patient.

10. The method of claim 9 wherein the patient geometry information for the particular patient comprises images;
and optionally:
wherein the patient geometry information for the particular patient comprises only images.

11. The method of claim 10 wherein the patient geometry information for the particular patient comprises images depicting at least one segmented and contoured organ-at-risk and at least one segmented and contoured planning target volume.

12. The method of any one of claims 9 to 11 wherein the at least one variable that is unrelated to the particular patient comprises a vector of random numerical input.

13. The method of any one of claims 9 to 12 wherein the therapeutic radiation delivery field geometry that is generated for the particular patient comprises, at least in part, field delivery directions.

14. The method of any one of claims 9 to 13 further comprising:
by the control circuit:
preprocessing the patient geometry information for the particular patient to thereby provide the information corresponding to the patient geometry information for the particular patient;
and optionally:
wherein preprocessing the patient geometry information comprises, at least in part, reducing dimensionality of the patient geometry information.

15. The method of claim 14 wherein the patient geometry information comprises, at least in part, a multidimensional numerical representation corresponding to an aggregation of different modalities of informational content;
and optionally:
wherein the aggregation of different modalities of informational content include, but are not limited to, imagery and non-imagery content.

## Patentansprüche

1. Vorrichtung zur Erleichterung des Erzeugens von Feldgeometrieinformationen für die Abgabe therapeutischer Strahlung für einen bestimmten Patienten, wobei die Vorrichtung Folgendes umfasst:
einen Speicher, in dem Patientengeometrieinformationen für den bestimmten Patienten gespeichert sind;
eine Steuerschaltung, die mit dem Speicher wirkverbunden und dazu konfiguriert ist:
- auf Informationen zuzugreifen, die den Patientengeometrieinformationen für den bestimmten Patienten entsprechen;
- mindestens eine Variable zu empfangen, die keinen Bezug zu dem bestimmten Patienten aufweist;
- die Informationen, die den Patientengeometrieinformationen für den bestimmten Patienten entsprechen, und die mindestens eine Variable als Eingabe für einen Feldgeometriegenerator bereitzustellen, wobei der Feldgeometriegenerator ein neuronales Netz umfasst, das in einem Framework bedingter generativer adversarischer Netze, GAN, als Funktion zuvor entwickelter Feldgeometrielösungen für eine Mehrzahl von verschiedenen Patienten trainiert ist, und wobei die Informationen, die den Patientengeometrieinformationen für den bestimmten Patienten entsprechen, als bedingte Eingabe für das neuronale Netz dienen;
- die Eingabe unter Verwendung des Feldgeometriegenerators zu verarbeiten, um dadurch die Feldgeometrie für die Abgabe therapeutischer Strahlung für den bestimmten Patienten zu erzeugen.

2. Vorrichtung nach Anspruch 1, wobei die Patientengeometrieinformationen für den bestimmten Patienten Bilder umfassen;
und optional:
wobei die Patientengeometrieinformationen für den bestimmten Patienten nur Bilder umfassen.

3. Vorrichtung nach Anspruch 1 oder 2, wobei die Patientengeometrieinformationen für den bestimmten Patienten Bilder umfassen, die mindestens ein segmentiertes und konturiertes Risikoorgan und mindestens ein segmentiertes und konturiertes Planungszielvolumen darstellen.

4. Vorrichtung nach einem der Ansprüche 1 bis 3, wobei die mindestens eine Variable, die keinen Bezug zu dem bestimmten Patienten aufweist, einen Vektor einer zufälligen numerischen Eingabe umfasst.

5. Vorrichtung nach einem der Ansprüche 1 bis 4, wobei die Feldgeometrie für die Abgabe therapeutischer Strahlung, die für den bestimmten Patienten erzeugt wird, zumindest teilweise Feldabgaberichtungen umfasst.

6. Vorrichtung nach einem der Ansprüche 1 bis 5, wobei die Steuerschaltung ferner dazu konfiguriert ist:
die Patientengeometrieinformationen für den bestimmten Patienten vorzuverarbeiten, um dadurch die Informationen bereitzustellen, die den Patientengeometrieinformationen für den bestimmten Patienten entsprechen.

7. Vorrichtung nach Anspruch 6, wobei die Steuerschaltung dazu konfiguriert ist, die Patientengeometrieinformationen zumindest teilweise durch Reduzieren der Dimensionalität der Patientengeometrieinformationen vorzuverarbeiten.

8. Vorrichtung nach Anspruch 7, wobei die Patientengeometrieinformationen zumindest teilweise eine mehrdimensionale numerische Darstellung umfassen, die einer Aggregation verschiedener Modalitäten von Informationsinhalt entspricht;
und optional:
wobei die Aggregation verschiedener Modalitäten von Informationsinhalt bildliche und nicht-bildliche Inhalte umfasst, ohne darauf beschränkt zu sein.

9. Verfahren zur Erleichterung des Erzeugens von Feldgeometrieinformationen für die Abgabe therapeutischer Strahlung für einen bestimmten Patienten, wobei das Verfahren Folgendes umfasst:
Bereitstellen eines Speichers, in dem Patientengeometrieinformationen für den bestimmten Patienten gespeichert sind;
Bereitstellen einer Steuerschaltung, die mit dem Speicher wirkverbunden ist;
durch die Steuerschaltung:
Zugreifen auf Informationen, die den Patientengeometrieinformationen für den bestimmten Patienten entsprechen;
Empfangen mindestens einer Variablen, die keinen Bezug zu dem bestimmten Patienten aufweist;
Bereitstellen der Informationen, die den Patientengeometrieinformationen für den bestimmten Patienten entsprechen, und der mindestens einen Variablen als Eingabe für einen Feldgeometriegenerator, wobei der Feldgeometriegenerator ein neuronales Netz umfasst, das in einem Framework bedingter generativer adversarischer Netze, GAN, als Funktion zuvor entwickelter Feldgeometrielösungen für eine Mehrzahl von verschiedenen Patienten trainiert ist, und
wobei die Informationen, die den Patientengeometrieinformationen für den bestimmten Patienten entsprechen, als bedingte Eingabe für das neuronale Netz dienen;
Verarbeiten der Eingabe unter Verwendung des Feldgeometriegenerators, um dadurch die Feldgeometrie für die Abgabe therapeutischer Strahlung für den bestimmten Patienten zu erzeugen.

10. Verfahren nach Anspruch 9, wobei die Patientengeometrieinformationen für den bestimmten Patienten Bilder umfassen;
und optional:
wobei die Patientengeometrieinformationen für den bestimmten Patienten nur Bilder umfassen.

11. Verfahren nach Anspruch 10, wobei die Patientengeometrieinformationen für den bestimmten Patienten Bilder umfassen, die mindestens ein segmentiertes und konturiertes Risikoorgan und mindestens ein segmentiertes und konturiertes Planungszielvolumen darstellen.

12. Verfahren nach einem der Ansprüche 9 bis 11, wobei die mindestens eine Variable, die keinen Bezug zu dem bestimmten Patienten aufweist, einen Vektor einer zufälligen numerischen Eingabe umfasst.

13. Verfahren nach einem der Ansprüche 9 bis 12, wobei die Feldgeometrie für die Abgabe therapeutischer Strahlung, die für den bestimmten Patienten erzeugt wird, zumindest teilweise Feldabgaberichtungen umfasst.

14. Verfahren nach einem der Ansprüche 9 bis 13, ferner umfassend:
durch die Steuerschaltung:
Vorverarbeiten der Patientengeometrieinformationen für den bestimmten Patienten, um dadurch die Informationen bereitzustellen, die den Patientengeometrieinformationen für den bestimmten Patienten entsprechen;
und optional:
wobei Vorverarbeiten der Patientengeometrieinformationen zumindest teilweise Reduzieren der Dimensionalität der Patientengeometrieinformationen umfasst.

15. Verfahren nach Anspruch 14, wobei die Patientengeometrieinformationen zumindest teilweise eine mehrdimensionale numerische Darstellung umfassen, die einer Aggregation verschiedener Modalitäten von Informationsinhalt entspricht;
und optional:
wobei die Aggregation verschiedener Modalitäten von Informationsinhalt bildliche und nicht-bildliche Inhalte umfasst, ohne darauf beschränkt zu sein.

## Revendications

1. Appareil pour faciliter la génération d'informations de géométrie de champ de délivrance de rayonnement thérapeutique pour un patient particulier, l'appareil comprenant :
une mémoire contenant des informations géométriques du patient pour le patient particulier ;
un circuit de commande couplé de manière fonctionnelle à la mémoire et configuré pour :
- accéder aux informations correspondant aux informations géométriques du patient pour le patient particulier ;
- recevoir au moins une variable qui n'est pas liée au patient particulier ;
- fournir en entrée à un générateur de géométrie de champ les informations correspondant aux informations géométriques du patient pour le patient particulier et à l'au moins une variable, dans lequel le générateur de géométrie de champ comprend un réseau neuronal entraîné dans un cadre de réseaux antagonistes génératifs conditionnels, GAN, en fonction de solutions de géométrie de champ précédemment développées pour une pluralité de patients différents et dans lequel les informations correspondant aux informations géométriques du patient pour le patient particulier servent d'entrée conditionnelle au réseau neuronal ;
- traiter l'entrée à l'aide du générateur de géométrie de champ pour ainsi générer la géométrie de champ de délivrance de rayonnement thérapeutique pour le patient particulier.

2. Appareil selon la revendication 1, dans lequel les informations géométriques du patient pour le patient particulier comprennent des images ;
et éventuellement :
dans lequel les informations géométriques du patient pour le patient particulier ne comprennent que des images.

3. Appareil selon la revendication 1 ou 2, dans lequel les informations géométriques du patient pour le patient particulier comprennent des images représentant au moins un organe à risque segmenté et délimité et au moins un volume cible de planification segmenté et délimité.

4. Appareil selon l'une quelconque des revendications 1 à 3, dans lequel l'au moins une variable qui n'est pas liée au patient particulier comprend un vecteur d'entrée numérique aléatoire.

5. Appareil selon l'une quelconque des revendications 1 à 4, dans lequel la géométrie de champ de délivrance de rayonnement thérapeutique qui est générée pour le patient particulier comprend, au moins en partie, des directions de délivrance de champ.

6. Appareil selon l'une quelconque des revendications 1 à 5, dans lequel le circuit de commande est en outre configuré pour :
prétraiter les informations géométriques du patient pour le patient particulier pour ainsi fournir les informations correspondant aux informations géométriques du patient pour le patient particulier.

7. Appareil selon la revendication 6, dans lequel le circuit de commande est configuré pour prétraiter les informations géométriques du patient, au moins en partie, en réduisant la dimensionnalité des informations géométriques du patient.

8. Appareil selon la revendication 7, dans lequel les informations géométriques du patient comprennent, au moins en partie, une représentation numérique multidimensionnelle correspondant à une agrégation de différentes modalités de contenu informationnel ;
et éventuellement :
dans lequel l'agrégation de différentes modalités de contenu informationnel comporte, mais sans s'y limiter, du contenu d'imagerie et du contenu sans imagerie.

9. Procédé pour faciliter la génération d'informations de géométrie de champ de délivrance de rayonnement thérapeutique pour un patient particulier, le procédé comprenant :
la fourniture d'une mémoire ayant des informations géométriques du patient pour le patient particulier ;
la fourniture d'un circuit de commande couplé de manière fonctionnelle à la mémoire ;
par le circuit de commande :
l'accès aux informations correspondant aux informations géométriques du patient pour le patient particulier ;
la réception d'au moins une variable qui n'est pas liée au patient particulier ;
la fourniture en entrée à un générateur de géométrie de champ des informations correspondant aux informations géométriques du patient pour le patient particulier et à l'au moins une variable, dans lequel le générateur de géométrie de champ comprend un réseau neuronal entraîné dans un cadre de réseaux antagonistes génératifs conditionnels, GAN, en fonction de solutions de géométrie de champ précédemment développées pour une pluralité de patients différents et dans lequel les informations correspondant aux informations géométriques du patient pour le patient particulier servent d'entrée conditionnelle au réseau neuronal ;
le traitement de l'entrée à l'aide du générateur de géométrie de champ pour ainsi générer la géométrie de champ de délivrance de rayonnement thérapeutique pour le patient particulier.

10. Procédé selon la revendication 9, dans lequel les informations géométriques du patient pour le patient particulier comprennent des images ;
et éventuellement :
dans lequel les informations géométriques du patient pour le patient particulier ne comprennent que des images.

11. Procédé selon la revendication 10, dans lequel les informations géométriques du patient pour le patient particulier comprennent des images représentant au moins un organe à risque segmenté et délimité et au moins un volume cible de planification segmenté et délimité.

12. Procédé selon l'une quelconque des revendications 9 à 11, dans lequel l'au moins une variable qui n'est pas liée au patient particulier comprend un vecteur d'entrée numérique aléatoire.

13. Procédé selon l'une quelconque des revendications 9 à 12, dans lequel la géométrie de champ de délivrance de rayonnement thérapeutique qui est générée pour le patient particulier comprend, au moins en partie, des directions de délivrance de champ.

14. Procédé selon l'une quelconque des revendications 9 à 13, comprenant en outre :
par le circuit de commande :
le prétraitement des informations géométriques du patient pour le patient particulier pour ainsi fournir les informations correspondant aux informations géométriques du patient pour le patient particulier ;
et éventuellement :
dans lequel le prétraitement des informations géométriques du patient comprend, au moins en partie, la réduction de la dimensionnalité des informations géométriques du patient.

15. Procédé selon la revendication 14, dans lequel les informations géométriques du patient comprennent, au moins en partie, une représentation numérique multidimensionnelle correspondant à une agrégation de différentes modalités de contenu informationnel ;
et éventuellement :
dans lequel l'agrégation de différentes modalités de contenu informationnel comporte, mais sans s'y limiter, du contenu d'imagerie et du contenu sans imagerie.
